## Europäisches Patentamt
## European Patent Office
## Office européen des brevets

(19)

(11) Publication number: **0 036 733**
**B1**

(12) **EUROPEAN PATENT SPECIFICATION**

(45) Date of publication of patent specification: **04.07.84**

(21) Application number: **81301086.5**

(22) Date of filing: **16.03.81**

(51) Int. Cl.³: **C 07 C 7/05, C 07 C 9/06, C 07 C 9/08, C 07 C 11/04**

(54) Distillative separation of carbon dioxide from light hydrocarbons.

(30) Priority: **18.03.80 US 131416**

(43) Date of publication of application:
**30.09.81 Bulletin 81/39**

(45) Publication of the grant of the patent:
**04.07.84 Bulletin 84/27**

(84) Designated Contracting States:
**AT BE CH DE FR GB IT LI NL SE**

(56) References cited:
**CA - A - 1 033 952**
**GB - A - 541 572**
**US - A - 2 744 394**
**US - A - 3 236 057**
**US - A - 4 246 015**
**US - A - 4 311 495**

(73) Proprietor: **KOCH PROCESS SYSTEMS, INC.**
**20 Walkup Drive**
**Westborough, MA 01581 (US)**

(72) Inventor: **Holmes, Arthur Sherwood**
**5 Olde Colony Drive**
**Shrewsbury Massachusetts 01545 (US)**
Inventor: **Ryan, James McKee**
**11 King's Grant Road**
**Weston Massachusetts 02193 (US)**

(74) Representative: **Holdcroft, James Gerald, Dr. et al,**
**Graham Watt & Co. Riverhead**
**Sevenoaks Kent TN13 2BN (GB)**

Courier Press, Leamington Spa, England.

## Description

This invention is in the field of distillation.

It is often desirable to separate acid gas components from light hydrocarbons in the processing of gas streams. Certain of these separations are, however, made difficult because of the tendency of mixtures of light hydrocarbons and acid gases to form azeotropes.

One such example can be found in the cryogenic distillative separation of methane from acid gas components described in our European Patent Application, No. 29 324. This process is effective for separating methane from high $CO_2$-content feed in one distillation column without solids formation. The bottoms product of this process contains carbon dioxide, ethane, and higher hydrocarbons, and it is often desirable to separate the carbon dioxide and ethane components in this bottoms product. Such a separation would produce a useful carbon dioxide product as well as an enriched ethane product which could be used for its heating value or as raw material in many chemical syntheses.

Although desirable, the separation of carbon dioxide from ethane by distillation has proven to be a difficult problem in practice. This difficulty is caused by the fact that carbon dioxide and ethane form an azeotrope of approximately two thirds carbon dioxide and one third ethane on a mole basis. For a feed mixture containing ethane and carbon dioxide, this azeotrope tends to form in the upper portion of the column, usually making further separation beyond the azeotrope composition impossible. The practice of employing two distillation towers operating at different pressures to work around the azeotrope does not help with the carbon dioxide/ethane system because pressure has only minimal effect on the composition of the azeotrope. Because of this, attempts to separate carbon dioxide from ethane by distillation have heretofore resulted in an overhead carbon dioxide stream containing approximately azeotropic amounts of ethane, which are unacceptable in many applications.

Ethylene also forms an azeotrope with carbon dioxide. This and other possible azeotropes between carbon dioxide and light hydrocarbons present limitations similar to those described for the carbon dioxide/ethane system when efforts are made to perform distillative separations on such systems.

The invention provides a distillative method of separating in distillation apparatus a mixture containing carbon dioxide and a light hydrocarbon into a carbon dioxide rich overhead fraction and a light hydrocarbon rich fraction the conditions of distillation being such that said carbon dioxide and light hydrocarbon are liable to form an azeotrope therebetween, characterised in that an additional liquid component is added to the mixture under distillation at a point above the feed to the distillation apparatus, which liquid component significantly alters the relative volatility between said carbon dioxide and said light hydrocarbon so that the volatility of the carbon dioxide is maintained above that of the light hydrocarbon at all points at and below the point of introduction of said liquid component, whereby the concentration of carbon dioxide in the overhead fraction exceeds the azeotropic composition.

The light hydrocarbon is for example ethane or ethylene.

The additional component comprises for example a $C_3$—$C_6$ alkane e.g. n-butane or a mixture of $C_3$—$C_6$ alkanes.

The invention also provides a method of separating a carbon dioxide rich overhead fraction from a mixture containing carbon dioxide and a light hydrocarbon which is ethane, characterised by the steps of: (a) introducing said mixture into a distillation column containing a plurality of vapor-liquid contact stages; (b) operating said distillation column at conditions of pressure, temperature and composition sufficient to produce an enriched carbon dioxide overhead stream and an enriched ethane bottoms stream, said conditions of temperature, pressure and composition also being liable to form a carbon dioxide/ethane azeotrope which would limit the concentration of carbon dioxide in said overhead stream to the azeotropic concentration, based upon a binary mixture of carbon dioxide and ethane; and (c) introducing into an upper portion of said distillation column at a point above the feed to the column a $C_3$—$C_6$ alkane or a mixture of $C_3$—$C_6$ alkanes as an additional liquid component which is miscible with the carbon dioxide and the ethane and significantly alters the relative volatility between said carbon dioxide and ethane so that the volatility of the carbon dioxide is maintained above that of the ethane at all points at and below the point of introduction of said liquid component, whereby the concentration of carbon dioxide in the overhead fraction exceeds the binary azeotropic composition.

A method embodying the present invention is essentially one of extractive distillation, which *per se* is a known technique; see for example "Elements of Fractional Distillation" by Robinson and Gilliland, McGraw Hill, 1950 Edition, and Perry's "Chemical Engineer's Handbook", McGraw Hill, 1953 Edition. Also, Canadian Patent No. 1,033,952 of Valdes discloses the specific application of extractive distillation to separation of hydrogen sulfide from a mixture with a light hydrocarbon employing e.g. butane as the extraction agent; but for carbon dioxide separation the Canadian patent proposes absorption techniques, rather than distillation.

However despite the existence of this prior art, and the long-standing problem of efficiently separating carbon dioxide from a light hydrocarbon, extractive distillation has not been previously proposed for breaking the carbon dioxide/light hydrocarbon azeotrope. If only a little carbon dioxide is present then straight-forward (non-extractive) distillation can be employed. Often, for mixtures

2

containing large amounts of carbon dioxide, non-distillative methods employing expensive physical solvents such as ethanolamines have been used.

Embodiments of the invention are described hereinafter by way of example. These embodiments involve the distillative separation of carbon dioxide from light hydrocarbons in mixtures wherein such separations are normally limited by the tendency to form an azeotrope. The feed mixtures can, of course, contain additional components e.g. higher hydrocarbons, nitrogen and hydrogen, etc.

In a method embodying this invention, a distillation column is used to separate carbon dioxide and light hydrocarbon, both present in the feed, into an overhead and bottoms product which are not limited by the azeotrope composition of the acid gas or light hydrocarbon. This is achieved by adding to the distillation column a component which prevents formation of the acid gas/light hydrocarbon azeotrope; the component is miscible in the liquid phase with the carbon dioxide and light hydrocarbon. For example, a component which prevents azeotrope formation can be employed in the distillative separation of a binary of carbon dioxide and ethane to produce an overhead stream having significantly more than two thirds carbon dioxide, which is the limitation when an azeotrope forms.

Extractive agents for extractive distillation have generally in the past been high-priced polar chemicals, and this is in contrast to the relatively simple inexpensive agents such as $C_3$—$C_6$ alkanes which can be used in the method embodying the present invention to produce high pressure carbon dioxide of a high degree of purity.

This method also has other inherent advantages. In many instances, for example, the energy requirements are lower than in the corresponding separation without the additional component because the relative volatility is improved or because the separation can be made at higher temperatures. This, of course, can lower the cost of the separation.

Sometimes materials capable of eliminating azeotropes are contained in the feed mixtures. For example, natural gas typically contains butane, which can be an effective agent for preventing azeotrope formation between carbon dioxide and ethane. Although the mere presence of such materials in the feed is not in itself sufficient to prevent azeotrope formation, such material can be separated from bottoms product and added back to the column at an appropriate point to prevent azeotrope formation. This takes advantage of materials already present in the feed as a convenient source of the additional component.

There now follows a description, to be read with reference to the accompanying drawings of embodiments of the invention. This description is given by way of example only, and not by way of limitation of the invention.

In the accompanying drawings:

Figure 1 is a plot of vapor-liquid equilibrium data for a binary system of carbon dioxide and ethane at two different pressures, as well as data from a computer simulated separation illustrating the effect on such a system of the addition of a component for preventing azeotrope formation;

Figure 2 is a plot of the relative volatility of carbon dioxide to ethane at various temperatures in a computer simulated distillation having a multi-component hydrocarbon mixture added to serve as a component for preventing azeotrope formation;

Figure 3 is a schematic flow diagram illustrating apparatus suitable for carrying out a method embodying the invention.

Much of the data presented in the following description, as well as that shown in the Figures, was obtained using a plate-to-plate column calculation program to simulate conditions within a distillation column for certain given or desired operating conditions. Unless otherwise stated, the program employed was the Process Simulation Program of Simulation Sciences, Inc., Fullerton, California, Oct.-Nov., 1979. Vapor-liquid equilibria and thermodynamic data were calculated based upon the Soave-Redlich-Kwong equation of state. While the total accuracy of the data obtained cannot be assured, and in fact will change somewhat depending upon the constants chosen, the data is believed to be representative of actual data and is certainly appropriate for illustrating and substantiating the benefits gained by use of a component to prevent azeotrope formation in distillative separations of an acid gas from a light hydrocarbon. For purposes of simplifying the plots, data from systems which were not binary were plotted on a pseudo-binary basis in which mole fractions are calculated as if the components beyond those in the binary were not present.

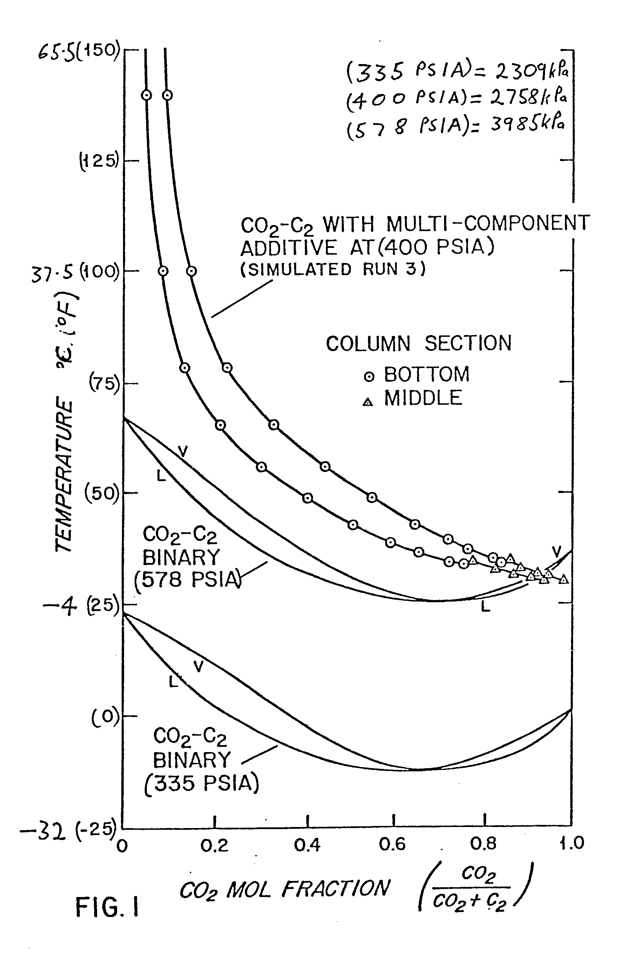

The practical difficulty of obtaining a complete separation of carbon dioxide from ethane in a gas mixture containing both can be seen by referring to Figure 1. Figure 1 contains vapor-liquid equilibria data for a binary mixture of carbon dioxide and ethane at both 578 psia and 335 psia. Data for these binary mixtures were obtained by Arthur D. Little, Inc. using an in-house computer program and employing the Soave-Redlich-Kwong equation of state. As can be seen from the data, the binary mixtures form a minimum boiling point azeotrope at both pressures in the range of about 65—70% carbon dioxide. Thus, a mixture of carbon dioxide and ethane tends toward the azeotropic amount as the gas rises up the distillation column. Once the azeotrope composition is reached, further separation of the binary does not occur. Thus, the overhead product typically contains the azeotropic amount of ethane, which in this case would amount to approximately one third of the overhead product, on a mole basis. The data also demonstrate that the effect of pressure on composition of an azeotrope in this

3

system is minimal, meaning that the use of two distillation columns operating at different pressures is not a viable solution to the problem.

The beneficial effect of adding a component to eliminate the azeotrope is demonstrated in the upper plot of vapor-liquid equilibria data for carbon dioxide and ethane, but additionally including a mixture of $C_3$—$C_6$ alkanes which together function as an additive to prevent azeotrope formation between carbon dioxide and ethane. The exact compositions and operating conditions are discussed in more detail below with reference to Simulated Run 3.

It can be seen from the data plotted that the azeotrope has been eliminated in the simulated run with additive. Thus, further separation of carbon dioxide and ethane, beyond the azeotrope amounts of the binary, can be achieved.

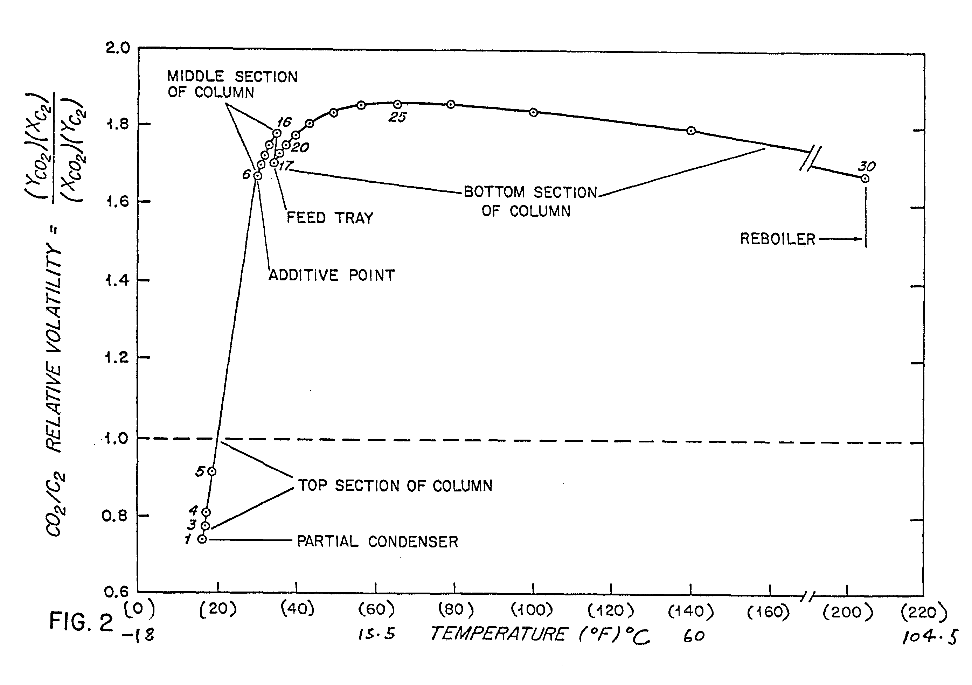

The beneficial effect of the additive is also dramatically demonstrated by the data regarding the relative volatility of carbon dioxide to ethane which is plotted in Figure 2. As seen in Figure 2, the relative volatility is about 1.65 at the point in the column where the additive is introduced. It remains at or above this value at all points below the point of addition, which comprise the middle and bottom sections of the column. In the top section of the column, above the additive addition point, the relative volatility slips to values below 1, indicating that ethane is actually more volatile than carbon dioxide in the top section of the column above where the additive was introduced. Thus, efforts to recover the additive actually reverse the natural relative volatilities of these materials.

The results of three distillations simulated on a computer will now be presented.

Simulated run 1

Summary

| | |
|---|---|
| Feed, mols[e]/hr | 1646 |
| Feed $CO_2$, mol % | 65 |
| Feed ethane, mol % | 9.9 |
| Stages[a], total | 20 |
| Additive rate, mols/hr | 986.3 |
| Ethane recovery in bottoms product, % | 64.7 |
| Ethane composition in overhead, mol % | 5.3 |
| $CO_2$ recovery in overhead, % | 96.8 |
| Additive loss into overhead, mols/hr | 2.5 |
| "Split", $\dfrac{(CO_2 \text{ Ohd})(\text{ethane Btm})}{(CO_2 \text{ Btm})(\text{ethane Ohd})}$ | 52.9 |

Column operation—3447 kPa (500 psia)

| Heating (cooling) Duty kw (MMBTU/hr) | | Temperature | Location stage | |
|---|---|---|---|---|
| (1407)[b](4.8) | (850)[c](2.9) | −1°C (30°F) | 1 | Overhead partial condenser |
| | | | 2 thru 5 | Top section of column |
| — | — | 37°C(98°F) | 6 | Additive fed in |
| | | | 7 thru 11 | Middle section of column |
| — | — | 20°C(68°F) | 12 | Feed |
| — | — | | 13 thru 19 | Bottom section of column |
| 5422(18.5) | 3282(11.2) | 122°C(251°F) | 20 | Reboiler |

Material balance

| | | Feed | Additive | Overhead | Bottoms | |
|---|---|---|---|---|---|---|
| Mols/hr $CO_2$ | | 1072.3 | 1.0 | 1038.0 | 35.3 | 96.8% recovery in overhead |
| Mols/hr ethane | | 163.5 | 1.0 | 58.7 | 105.8 | 64.7% recovery in bottoms |
| Mols/hr propane | | 142.2 | 1.0 | .2 | 143.0 | |
| Mols/hr n-butane | | 268.5 | 963.3 | 2.3 | 1249.5 | |
| | Total | 1646.5 | 986.3 | 1099.2 | 1533.6 | |
| Mol % $CO_2$ | | 65.1 | 0.1 | 94.4 | 2.3 | |
| Mol % ethane | | 9.93 | 0.1 | 5.3 | 6.9 | |
| Mol % propane-plus | | 25.0 | 99.8 | 0.23 | 90.8 | |
| °C (°F) temperature | | 17 (62.9) | 31 (88.0) | −1 (30.3) | 122 (251.4) | |
| Mol % liquid | | 100 | 100 | 0 | 100 | |
| Mol % $CO_2$ (pseudo-bin.[d]) | | 86.8 | — | 94.6 | 25.0 | |

4

[a] Stages are theoretical stages (trays, partial condensers, reboilers), 100% efficient.
[b] For full amount of feed, as shown.
[c] Per 1000 mols/hr of feed.

[d] Pseudo-binary $CO_2$ composition = $\left(\dfrac{CO_2}{CO_2 + \text{ethane}}\right) \times 100$.

[e] Mols cited are: 1 lb mol = 0.454 kg mol.

## Conditions at selected stages

| Stage number | 1 | 6 | 12 | 20 |
|---|---|---|---|---|
| Temperature, °C (°F) | −1 (30.3) | 37 (98.0) | 20 (67.6) | 122 (251) |
| Liquid, $CO_2$, mol % | 94.7 | 35.6 | 46.7 | 2.30 |
| Liquid, ethane, mol % | 4.45 | 4.23 | 10.1 | 6.89 |
| Liquid, propane-plus, mol % | 0.85 | 60.2 | 43.2 | 90.8 |
| Liquid, reduced temp. | 0.892 | 0.822 | 0.828 | 0.966 |
| Liquid flow, mols/hr | 1100 | 2093 | 3634 | 1534 |
| Vapor, $CO_2$, mol % | 94.4 | 82.1 | 81.5 | 5.86 |
| Vapor, ethane, mol % | 5.34 | 5.34 | 10.4 | 12.8 |
| Vapor, propane-plus, mol % | 0.23 | 12.6 | 8.1 | 81.3 |
| Vapor flow, mols/hr | 1099 | 1803 | 2355 | 3510 |
| Relative volatility, $CO_2$: ethane | 0.831 | 1.83 | 1.69 | 1.37 |

Simulated run 2

Summary

| | |
|---|---|
| Feed, mols[e]/hr | 9945 |
| Feed $CO_2$, mol % | 32 |
| Feed ethane, mol % | 19 |
| Stages[a], total | 25 |
| Additive rate, mols/hr | 6087 |
| Ethane recovery in bottoms product, % | 91.0 |
| Ethane composition in overhead, mol % | 5.1 |
| $CO_2$ recovery in overhead, % | 95.9 |
| $H_2S$ elimination from overhead, % | 98.3 |
| Additive loss into overhead, mols/hr | 7.8 |

"Split", $\dfrac{(CO_2 \text{ Ohd})(\text{ethane Btm})}{(CO_2 \text{ Btm})(\text{ethane Ohd})}$      234

## Column operation—2758 kPa (400 psia).

| Heating (cooling) Duty kw (MMBTU/hr) | | Temperature | Location stage | |
|---|---|---|---|---|
| [b] | [c] | | | |
| (3195) (10.9) | (322) (1.1) | −9.5°C (15°F) | 1 | Overhead partial condenser |
| | | | 2 thru 5 | Top section of column |
| (4015) (13.7) | (410) (1.4) | 16.5°C (62°F) | 6 | Intermediate partial condenser Additive added to stage 6 |
| | | | 7 thru 13 | Middle section of column |
| — | — | 26.5°C (80°F) | 14 | Feed vapor addition |
| — | — | 26.5°C (80°F) | 15 | Feed liquid addition |
| | | | 16 thru 24 | Bottom section of column |
| 27.550 (94.0) | 2755 (9.4) | 99°C (210°F) | 25 | Reboiler |

Material balance

| | Feed | Additive | $CO_2$ product overhead | Ethane-plus product bottoms | |
|---|---|---|---|---|---|
| Mols/hr methane | 32.1 | 0.0 | 0.0 | 32.1 | 95.9% recovery in overhead |
| Mols/hr $CO_2$ | 3189.8 | 0.0 | 3058.1 | 131.7 | 91.0% recovery in bottoms |
| Mols/hr ethane | 1851.7 | 0.0 | 167.0 | 1684.7 | 98.3% elimination from overhead |
| Mols/hr $H_2S$ | .235 | 0.0 | 0.0040 | .231 | |
| Mols/hr propane | 1026.0 | 60.9 | 2.8 | 1084.1 | |
| Mols/hr butane-plus | 3844.8 | 6025.7 | 5.0 | 9865.5 | |
| Total | 9944.6 | 6086.6 | 3232.9 | 12798.3 | |
| Mol % $CO_2$ | 32.1 | 0.0 | 93.7 | 1.03 | |
| Mols % $C_2$ | 18.6 | 0.0 | 5.11 | 13.2 | |
| Mol % propane-plus | 49.0 | 100.0 | .24 | 85.8 | |
| ppm $H_2S$ | 23.6 | 0.0 | 1.22 | 18.1 | |
| °C (°F) temp. | 24.5 (75.8) | 14 (57.0) | −9.5 (14.8) | 99 (209.5) | |
| Mol % liquid | 83.6 | 100.0 | 0.0 | 100.0 | |
| Mol % $CO_2$ (pseudo-bin[a]) | 63.3 | — | 94.8 | 7.3 | |

[a]Stages are theoretical stages (trays, partial condensers, reboilers), 100% efficient.
[b]For full amount of feed, as shown.
[c]Per 1000 mols/hr. of feed.

[d]Pseudo-binary $CO_2$ composition$= \left( \dfrac{CO_2}{CO_2 + ethane} \right) \times 100.$

[e]Mols cited are lb-mols: 1 lb mol=0.454 kg mol.

Conditions at selected stages

| Stage number | 1 | 6 | 15 | 25 |
|---|---|---|---|---|
| Temperature, °C (°F) | −9.5 (14.8) | 16.5 (61.9) | 26.5 (79.5) | 99 (210) |
| Liquid, $CO_2$, mol % | 95.1 | 39.3 | 25.1 | 1. |
| Liquid, ethane, mol % | 4.01 | 4.46 | 18.3 | 13. |
| Liquid, propane-plus, mol % | 0.76 | 56.1 | 56.5 | 85. |
| Liquid, reduced temperature | 0.865 | 0.780 | 0.811 | 0. |
| Liquid flow, mols/hr | 2250 | 12173 | 21238 | 1276 |
| Vapour, $CO_2$, mols/hr | 93.7 | 86.4 | 63.4 | 3. |
| Vapor, ethane, mol % | 5.11 | 5.10 | 24.2 | 30. |
| Vapor, propane-plus, mol % | 0.24 | 7.8 | 12.1 | 66. |
| Vapor flow, mols/hr | 3265 | 4930 | 8395 | 1323 |
| Relative volatility $CO_2$: ethane | 0.773 | 1.92 | 1.91 | 1. |

Simulated run 3
Summary

| | |
|---|---|
| Feed, mols[e]/hr | 1776 |
| Feed $CO_2$ mol % | 60 |
| Feed ethane, mol % | 18 |
| Stages[a]: total | 30 |
| Additive rate, mols/hr | 995 |
| Ethane recovery in bottoms product | 93.5 |
| Ethane, composition in overhead, mol % | 1.8 |
| $CO_2$ recovery in overhead, % | 99.0 |
| $H_2S$ elimination from overhead, % | 99.8 |
| Additive loss into overhead, mols/hr | 3.9 |
| "Split", $\dfrac{(CO_2 \text{ Ohd) (Ethane Btm)}}{(CO_2 \text{ Btm) (ethane Ohd)}}$ | 1410 |

Column operating conditions—2758 kPa (400 psia)

| Heating (cooling) Duty kw (MMBTU/hr) | | Temperature | Location stage | |
|---|---|---|---|---|
| (1128)[a] (3.85) | (645)[b] (2.2) | −9°C (16°F) | 1 | Overhead partial condenser |
| | | | | Top section of column |
| (1594) (5.44) | (909) (3.1) | −1°C (30°F) | 2 thru 5 | Intermediate partial condenser |
| | | | | Additive added to 6th stage |
| | | | | Middle section of column |
| — | — | 1°C (34°F) | 7 thru 16 | Feed addition |
| | | | | Bottom section of column |
| 6741 (23.0) | 3810 (13.0) | 96°C (205°F) | 18 thru 25 | Reboiler |

Material balance

| | Feed | Additive | CO$_2$ product overhead | Ethane-plus product bottoms | |
|---|---|---|---|---|---|
| Mols/hr CO$_2$ | 1069.0 | 0.0 | 1058.1 | 10.9 | 99.0% Recovery in Ohd |
| Mols/hr ethane | 310.0 | 0.0 | 20.0 | 290.0 | 93.5% Recovery in Btm |
| Mols/hr H$_2$S | 5.0 | 0.0 | 0.0083 | 4.997 | 99.8% Elimin. from Ohd |
| Mols/hr propane | 253.0 | 59.7 | 3.2 | 309.5 | |
| Mols/hr n-butane | 92.0 | 463.6 | 0.7 | 554.9 | |
| Mols/hr n-pentane | 36.0 | 362.1 | 0.0 | 398.1 | |
| Mols/hr n-hexane | 11.0 | 109.4 | 0.0 | 120.4 | |
| Total | 1776.0 | 994.8 | 1082.0 | 1688.8 | |
| Mol % CO$_2$ | 60.2 | 0.0 | 97.8 | 0.65 | |
| Mol % C$_2$ | 17.5 | 0.0 | 1.8 | 17.2 | |
| Mol % propane-plus | 22.1 | 100 | 0.36 | 81.9 | |
| ppm H$_2$S | 2800 | 0 | 7.7 | 3000 | |
| °C (°F) temp. | −0.5 (30.7) | −0.5 (31.0) | −8.5 (16.3) | 96 (204.6) | |
| Mol % liquid | 79.1 | 100.0 | 0.0 | 100.0 | |
| Mol % CO$_2$ (pseudo-bin[d]) | 77.5 | — | 98.1 | 3.6 | |

[a]Stages are theoretical stages (trays, partial condensers, reboilers), 100% efficient

[b]For full amount of feed, as shown.

[c]Per 1000 mols/hr, of feed

[d]Pseudo-binary CO$_2$ composition $= \left(\dfrac{CO_2}{CO_2 + \text{ethane}}\right) \times 100$

[e]Mols cited are lb-mols: 1 lb mol=0.454 kg mol.

Conditions at selected stages

| Stage number | 1 | 6 | 17 | 30 |
|---|---|---|---|---|
| Temperature, °C (°F) | −8.5 (16.3) | −1 (30.2) | 1.5 (34.4) | 96 (205.0) |
| Liquid, CO$_2$, Mol% | 97.9 | 65.5 | 51.9 | 0.64 |
| Liquid, Ethane, Mol% | 1.37 | 1.89 | 16.2 | 17.2 |
| Liquid, Propane-Plus, Mol% | 0.76 | 32.6 | 31.7 | 81.9 |
| Liquid, Reduced Temperature | 0.867 | 0.777 | 0.797 | 0.90 |
| Liquid Flow, Mols/Hr | 790 | 3313 | 4758 | 1689 |
| Vapor, CO$_2$, Mol% | 97.8 | 95.7 | 80.1 | 2.60 |
| Vapor, Ethane, Mol% | 1.85 | 1.66 | 14.9 | 41.2 |
| Vapor, Propane-Plus, Mol% | 0.37 | 2.67 | 4.15 | 55.6 |
| Vapor Flow, Mols/Hr | 1082 | 1829 | 3429 | 2874 |
| Relative Volatility CO$_2$: Ethane | 0.740 | 1.66 | 1.68 | 1.68 |

Since the azeotrope composition is between 65—70% carbon dioxide, the feeds presented in the three simulations of 87%, 63% and 78% (pseudo-binary basis) are on both the high and low sides of the azeotrope composition. The respective bottom products lean in carbon dioxide of 25%, 7% and 4% are significantly lower than the feed composition. The overhead products, rich in carbon dioxide at 95%, 95% and 98%, are significantly richer in carbon dioxide content than the azeotrope composition. Thus, the additive has made possible a more complete separation of carbon dioxide from ethane in all three simulations. A distillation without additive could not have produced such complete separations. Simulation 3 is particularly noteworthy in that the carbon dioxide overhead stream was substantially free of ethane and the ethane bottoms product was substantially free of carbon dioxide. Even more complete separations could be achieved.

The heating and cooling duties are tabulated per 1,000 moles/hour of feed. In this regard, Simulated Run 2 has the least consumption of heating and cooling whereas Simulated Run 3 has the greatest consumption of each.

In regard to the split function, Simulation Run 1 has the least thorough split whereas Simulated Run 3 has the most thorough split.

In general, any material or mixture of materials which causes the relative volatility of an acid gas to a light hydrocarbon from which it is to be separated by distillation to be significantly different than 1 over the range of interest is satisfactory as an additional component. Liquids which are miscible with the acid gas and light hydrocarbon, such as $C_3$—$C_6$ alkanes, are preferred because they are typically present in feed mixtures, are easy to separate and recycle, and often have a very beneficial effect in causing the acid gas to be more volatile relative to the light hydrocarbon. Natural gas liquids (NGL) contain such alkanes and can often be separated from bottoms product in conventional separation equipment. Thus, NGL or components thereof can be conveniently recycled to provide the additional component. It is also clear that materials satisfactory as the additional component need not be pure material. In general, the component should be liquid at the overhead conditions in the distillation column, and it is desirable to have components which have volatilities lower than the components to be separated. The additional component should also have a freezing point sufficiently low to avoid solids formation in the column.

In addition to the preferred materials mentioned above, there are other classes of materials which meet these requirements. For example, other hydrocarbons such as higher alkanes and naphthenes, halogenated hydrocarbons such as fluorochloromethane and fluoro-chloroethane compounds; and sulphur dioxide are believed to be suitable. Those skilled in the art will know, or be able to ascertain using no more than routine experimentation, other materials suitable as additional components.

The amount of additional component added will be dependent upon factors such as e.g. the composition of the feed, operating pressure, throughput of the column, recovery of overhead and bottoms product desired. Such factors can be taken into account by those skilled in the art by determining the operative amounts for any given separation using no more than routine experimentation.

The additional component is added to the column at a point above where feed is introduced since this is where azeotrope formation normally occurs. Although some materials which are suitable are contained in the feed in some cases, this alone is in the embodiments of the invention not sufficient to prevent azeotrope formation. This is because the additional component is usually not sufficiently volatile to rise up the column to the problem area. Thus, even if present in the feed, the component is separated and added to a point above the feed.

Although it is possible to add the additional component at the top of the column, including into the condenser, this is usually not desirable because the additional component cannot then be separated efficiently from overhead product. Thus, it is preferable to add the component in most cases at a point below the column top to thereby allow separation of additive from the desired overhead product.

In some cases, it is desirable to add the component at more than one column location on a simultaneous basis.

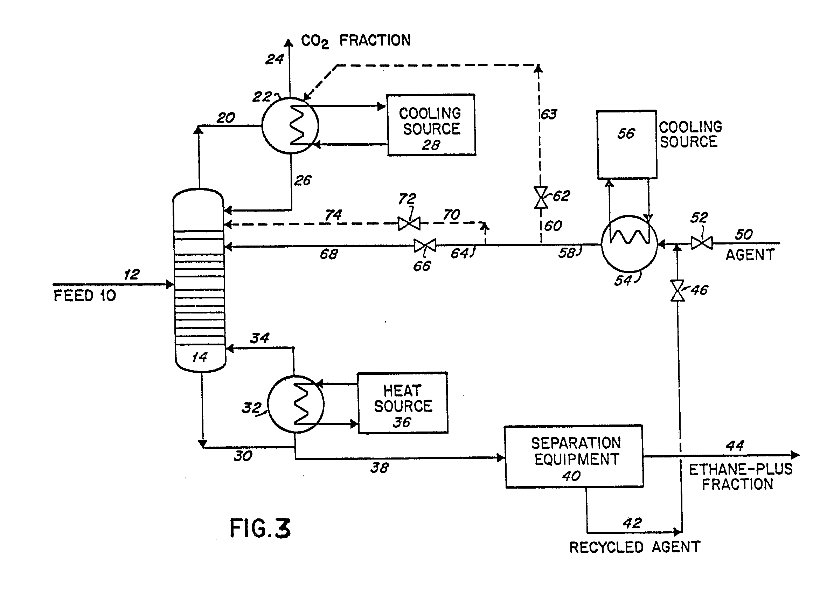

An apparatus for carrying out a separation of carbon dioxide from ethane is schematically illustrated in Figure 3. Therein, feed mixture 10, containing a mixture of carbon dioxide and ethane, and usually other components e.g. heavier hydrocarbons, and nitrogen, enters through feed line 12 into distillation column 14. Column 14 contains a number of vapor-liquid contact stages such as trays or packing, with the exact number of contact stages depending upon the required operating conditions.

Overhead stream 20 is rich in carbon dioxide and passes to partial condenser 22 from which the remaining vapor stream 24 exits as carbon dioxide product. This product stream also contains components present in the feed which are more volatile than carbon dioxide, such as any nitrogen present in the feed. Liquid from the partial condenser returns to column 14 in line 26 where it serves as reflux for tower 14. Condenser 22 is cooled by external cooling source 28.

The bottoms stream exits from the lower portion of column 14 in bottoms line 30 and contains ethane and other less volatile hydrocarbons or other components, and any component added to prevent azeotrope formation. A portion of the bottoms product is passed through reboiler 32 and back to column 14 in line 34. Reboiler 32 is heated by an external heat source 36.

The bottoms product passes in line 38 to further separation equipment 40, such as another distillation column. Separation equipment 40 is employed to separate out the additional component which is recycled in line 42 back to the column. The amount of recycled agent can be controlled by flow control valve 46. An ethane fraction is also separated in equipment 40 and is directed in line 44 to suitable ethane product facilities.

Component for preventing azeotrope formation may also be added to the system through line 50 and flow control valve 52. Such externally added agent may be used in lieu of recycled agent or in conjunction with recycled agent. In either case, the agent is cooled in heat exchanger 54, cooled by cooling source 56, and directed through flow lines 58 back towards the column 14.

The additional component can be added at a number of different locations, either individually or at several locations simultaneously. As illustrated, the component can be directed in line 64 to flow control valve 66 and flow line 68 and introduced directly onto a tray in the upper section of column 14 above the feed line 12. Similarly, the component can be added to a higher column tray, such as by passing it in line 70 through control valve 72 and line 74. The component can also be introduced into condenser 22 by directing it through line 60, flow control valve 62 and line 63. Other suitable points of addition can be determined for each particular separation being performed.

## Claims

1. A distillative method of separating in distillation apparatus (14) a mixture containing carbon dioxide and a light hydrocarbon into a carbon dioxide rich overhead fraction and a light hydrocarbon rich fraction the conditions of distillation being such that said carbon dioxide and light hydrocarbon are liable to form an azeotrope therebetween, characterised in that an additional liquid component is added to the mixture under distillation at a point (26, 74, 68) above the feed (12) to the distillation apparatus (14), which liquid component significantly alters the relative volatility between said carbon dioxide and said light hydrocarbon so that the volatility of the carbon dioxide is maintained above that of the light hydrocarbon at all points at and below the point (26, 74, 68) of introduction of said liquid component, whereby the concentration of carbon dioxide in the overhead fraction exceeds the azeotropic composition.

2. A method according to claim 1, wherein said light hydrocarbon is ethane or ethylene.

3. A method according to claim 1 or claim 2, wherein the additional component is already a component of the mixture to be separated, and, having been separated from the mixture, is recycled to the mixture under distillation.

4. A method according to claim 1 or claim 2, wherein the mixture to be separated comprises natural gas which contains butane, and the butane is separated from the natural gas and recycled to the mixture under distillation as the additional liquid component.

5. A method according to any one of the claims 1, 2 and 3, wherein said additional component comprises a $C_3$—$C_6$ alkane or a mixture of $C_3$—$C_6$ alkanes.

6. A method of separating a carbon dioxide rich overhead fraction from a mixture containing carbon dioxide and a light hydrocarbon which is ethane, characterised by the steps of: (a) introducing said mixture into a distillation column (14) containing a plurality of vapor-liquid contact stages; (b) operating said distillation column (14) at conditions of pressure, temperature and composition sufficient to produce an enriched carbon dioxide overhead stream (20) and an enriched ethane bottoms stream (30), said conditions of temperature, pressure and composition also being liable to form a carbon dioxide/ethane azeotrope which would limit the concentration of carbon dioxide in said overhead stream to the azeotropic concentration, based upon a binary mixture of carbon dioxide and ethane; and (c) introducing into an upper portion of said distillation column (14) at a point (26, 74, 68) above the feed (12) to the column (14) a $C_3$—$C_6$ alkane or a mixture of $C_3$—$C_6$ alkanes as an additional liquid component which is miscible with the carbon dioxide and the ethane and significantly alters the relative volatility between said carbon dioxide and ethane so that the volatility of the carbon dioxide is maintained above that of the ethane at all points at and below the point (26, 74, 68) of introduction of said liquid component, whereby the concentration of carbon dioxide in the overhead fraction exceeds the binary azeotropic composition.

7. A method according to any one of the preceding claims, wherein said additional liquid component is separated from bottoms fraction and recycled to the mixture under distillation.

8. A method according to claim 1 or claim 6, wherein said additional component comprises n-butane.

9. A method according to any one of the preceding claims, wherein overhead fraction is condensed (22) and refluxed to the mixture under distillation, and the additional component is introduced into said condensed overhead fraction and added to the mixture under distillation with the refluxed overhead fraction at the point (26) above the feed (12).

10. A method according to any one of the preceding claims, wherein the relative volatility of carbon dioxide to light hydrocarbon at and below the point where the additional component is introduced is at least 1.65.

## Patentansprüche

1. Verfahren zum destillativen Trennen eines Kohlendioxid und einen leichten Kohlenwasserstoff enthaltenden Gemisches in eine kohlendioxidreiche Kopffraktion und eine an leichtem Kohlenwasserstoff reiche Kopffraktion in einer Destilliervorrichtung (14) unter derartigen Destillierbedingungen, daß das Kohlendioxid und der leichte Kohlenwasserstoff dazu neigen, miteinander ein azeotropes Gemisch zu bilden, dadurch gekennzeichnet, daß eine zusätzliche Flüssigkomponente dem der Destillation unterworfenen Gemisch an einem Punkt (26, 74, 68) oberhalb des Zulaufs (12) zur Destilliervorrichtung (14) zugesetzt wird, wobei diese Flüssigkomponente die relative Flüchtigkeit zwischen dem Kohlendioxid und dem leichten Kohlenwasserstoff wesentlich verändert, derart, daß an allen Punkten am und unterhalb des Einführungspunktes (26, 74, 68) der Flüssigkomponente die Flüchtigkeit des Kohlendioxids oberhalb der des leichten Kohlenwasserstoffes gehalten wird, wobei die Konzentration von Kohlendioxid in der Kopffraktion die azeotrope Zusammensetzung übersteigt.

2. Verfahren nach Anspruch 1, dadurch gekennzeichnet, daß der leichte Kohlenwasserstoff Äthan oder Äthylen ist.

3. Verfahren nach Anspruch 1 oder 2, dadurch gekennzeichnet, daß die Zusatzkomponente bereits eine Komponente des zu trennenden Gemisches ist und, nach Abtrennen von dem Gemisch, dem der Destillation ausgesetzten Gemisch wieder zugeleitet wird.

4. Verfahren nach Anspruch 1 oder 2, dadurch gekennzeichnet, daß das zu trennende Gemisch Butan enthaltendes Erdgas umfaßt und das Butan vom Erdgas abgetrennt und dem der Destillation ausgesetzten Gemisch als zusätzliche Flüssigkomponente wieder zugeführt wird.

5. Verfahren nach einem der Ansprüche 1, 2 und 3, dadurch gekennzeichnet, daß die Zusatzkomponente ein $C_3$—$C_6$-Alkan oder ein Gemisch aus $C_3$—$C_6$-Alkanen umfaßt.

6. Verfahren zum Abtrennen einer kohlendioxidreichen Kopffraktion aus einem Gemisch aus Kohlendioxid und einem aus Äthan bestehenden leichten Kohlenwasserstoff, gekennzeichnet durch folgende Verfahrensstufen: (a) das Gemisch wird in eine Destillationskolonne (14) mit einer Mehrzahl von Dampf-Flüssigkeit-Berührungsstufen eingeführt; (b) die Destillationskolonne (14) wird unter derartigen Druck-, Temperatur- und Zusammensetzungsbedingungen betrieben, daß ein angereicherter Kohlendioxidkopfstrom (20) und ein angereicherter Äthanbodenstrom (30) erzeugt werden, wobei diese Druck-, Temperatur- und Zusammensetzungsbedingungen ferner zur Bildung eines azeotropen Gemisches aus Kohlendioxid und Äthan neigen, das die Konzentration des Kohlendioxids im Kopfstrom auf die azeotrope Konzentration auf der Basis eines Zweistoffgemisches aus Kohlendioxid und Äthan begrenzen würde; (c) in einen oberen Bereich der Destillationskolonne (14) an einem Punkt (26, 74, 68) oberhalb des Zulaufs (12) zur Kolonne (14) wird ein $C_3$—$C_6$-Alkan oder ein Gemisch von $C_3$—$C_6$-Alkanen als eine zusätzliche Flüssigkomponente eingeführt, die mit dem Kohlendioxid und dem Äthan mischbar ist und die relative Flüchtigkeit zwischen dem Kohlendioxid und dem Äthan wesentlich ändert, derart, daß an allen Punkten am oder unterhalb des Einführungspunktes (26, 74, 68) der Flüssigkomponente die Flüchtigkeit des Kohlendioxids oberhalb der des Äthans gehalten wird, wobei die Konzentration von Kohlendioxid in der Kopffraktion die azeotrope Zweistoffzusammensetzung übersteigt.

7. Verfahren nach einem der vorhergehenden Ansprüche, dadurch gekennzeichnet, daß die zusätzliche Flüssigkomponente von der Bodenfraktion abgetrennt und dem der Destillation ausgesetzten Gemisch wieder zugeführt wird.

8. Verfahren nach Anspruch 1 oder 6, dadurch gekennzeichnet, daß die Zusatzkomponente n-Butan umfaßt.

9. Verfahren nach einem der vorhergehenden Ansprüche, dadurch gekennzeichnet, daß die Kopffraktion kondensiert (22) und dem der Destillation ausgesetzten Gemisch wieder zugeführt wird und die Zusatzkomponente in die kondensierte Kopffraktion eingeführt und dem der Destillation ausgesetzten Gemisch mit der rückgeführten Kopffraktion an dem Punkt (26) oberhalb des Zulaufs (12) zugesetzt wird.

10. Verfahren nach einem der vorhergehenden Ansprüche, dadurch gekennzeichnet, daß die relative Flüchtigkeit von Kohlendioxid zu leichtem Kohlenwasserstoff am und unterhalb des Punktes, an dem die Zusatzkomponente zugeführt wird, zumindest 1,65 beträgt.

**Revendications**

1. Procédé de séparation par distillation dans an appareil de distillation (14) d'un mélange contenant du dioxyde de carbone et un hydrocarbure léger en une fraction de tête riche en dioxyde de carbone et une fraction riche en hydrocarbures légers, les conditions de distillation étant telles que le dioxyde de carbone et l'hydrocarbure léger sont susceptibles de former un azéotrope entre eux, caractérisé en ce qu'un composant liquide supplémentaire est ajouté au mélange sous distillation en un point (26, 74, 68) situé au-dessus du point (12) d'introduction d'une charge d'alimentation dans l'appareil (14) de distillation, lequel composant liquide modifie notablement la volatilité relative entre ledit dioxyde de carbone et ledit hydrocarbure léger en sorte que la volatilité du dioxyde de carbone est maintenue au-dessus de celle de l'hydrocarbure léger en tous les points situés au niveau du point (26, 74, 68) d'introduction dudit composant liquide, et au-dessous de ce point de manière que la concentration de dioxyde de carbone dans la fraction de tête dépasse la composition azéotropique.

2. Procédé selon la revendication 1, dans lequel ledit hydrocarbure léger est l'éthane ou l'éthylène.

3. Procédé selon la revendication 1 ou la revendication 2, dans lequel le composant supplémentaire est déjà un composant du mélange à séparer, et ayant été séparé du mélange, est recyclé dans le mélange sous distillation.

4. Procédé selon la revendication 1 ou la revendication 2, dans lequel le mélange à séparer comprend du gaz naturel qui contient du butane, et le butane est séparé du gaz naturel et recyclé dans le mélange sous distillation en tant que composant liquide supplémentaire.

5. Procédé selon l'une quelconque des revendications 1, 2 et 3, dans lequel ledit composant supplémentaire comprend un alcane en $C_3$—$C_6$ ou un mélange d'alcanes en $C_3$—$C_6$.

6. Procédé de séparation d'une fraction de tête riche en dioxyde de carbone d'un mélange contenant du dioxyde de carbone et un hydrocarbure léger qui est de l'éthane caractérisé par les étapes de: (a) introduction dudit mélange dans une colonne (14) de distillation contenant plusieurs étages de contact vapeur-liquide; (b) mise en action de ladite colonne (14) de distillation à des conditions de pression, de température et de composition suffisantes pour produire un courant de tête (20) enrichi en dioxyde de carbone et un courant de queue (30) enrichi en éthane, lesdites conditions de température, de pression et de composition étant également susceptibles de former un azéotrope dioxyde de carbone/éthane pouvant limiter la concentration de dioxyde de carbone dans ledit courant de tête à la concentration azéotropique, basée sur un mélange binaire de dioxyde de carbone et d'éthane; et (c) introduction dans une partie supérieure de ladite colonne (14) de distillation en un point (26, 74, 68) situé au-dessus du point (12) d'introduction de la charge dans la colonne (14) d'un alcane en $C_3$—$C_6$ ou d'un mélange d'alcanes en $C_3$—$C_6$ à titre de composant liquide supplémentaire qui est miscible au dioxyde de carbone et à l'éthane et modifie notablement la volatilité relative entre ledit dioxyde de carbone et l'éthane en sorte que la volatilité du dioxyde de carbone est maintenue au-dessus de celle de l'éthane en tous les points situés au niveau du point (26, 74, 68) d'introduction dudit composant liquide, ou au-dessous de ce point de manière que la concentration de dioxyde de carbone dans la fraction de tête dépasse la composition azéotropique binaire.

7. Procédé selon l'une quelconque des revendications précédentes, dans lequel ledit composant supplémentaire liquide est séparé de la fraction de queue et recyclé dans le mélange sous distillation.

8. Procédé selon la revendication 1 ou la revendication 6, dans lequel ledit composant supplémentaire comprend du n-butane.

9. Procédé selon l'une quelconque des revendications précédentes, dans lequel la fraction de tête est condensée (22) et recyclée dans le mélange sous distillation et le composant supplémentaire est introduit dans ladite fraction de tête condensée et ajouté au mélange sous distillation avec la fraction de tête recyclée au point (26) situé au-dessus du point (12) d'introduction de la charge.

10. Procédé selon l'une quelconque des revendications précédentes, dans lequel la volatilité relative du dioxyde de carbone et de l'hydrocarbure léger au point d'introduction du composant supplémentaire ou au-dessous de ce point, est d'au moins 1,65.

FIG. I

FIG. 2

FIG.3